(19) Europäisches Patentamt
European Patent Office
Office européen des brevets

(11) Numéro de publication: **0 169 148**
**B1**

# (12) FASCICULE DE BREVET EUROPÉEN

(45) Date de publication du fascicule du brevet: **13.09.89**

(51) Int. Cl.⁴: **C 07 D 401/04,** A 61 K 31/445

(21) Numéro de dépôt: **85401481.8**

(22) Date de dépôt: **18.07.85**

(54) **Nouveaux dérivés de l'indole, leur préparation, leur application comme médicaments et les compositions les renfermant.**

(30) Priorité: **19.07.84 FR 8411432**

(43) Date de publication de la demande: **22.01.86 Bulletin 86/4**

(45) Mention de la délivrance du brevet: **13.09.89 Bulletin 89/37**

(84) Etats contractants désignés: **BE CH DE FR GB IT LI LU NL**

(56) Documents cité:
**EP-A-0 021 924**
**EP-A-0 071 520**
**EP-A-0 071 521**
**EP-A-0 112 191**

(73) Titulaire: **ROUSSEL- UCLAF, 35, boulevard des Invalides, F-75007 Paris (FR)**

(72) Inventeur: **Guillaume, Jacques, 15, avenue du Belvédère (appt. 1904), F-93310 Le Pre Saint Gervais (FR)**
Inventeur: **Clemence, François, 2, rue Turgot, F-75009 Paris (FR)**
Inventeur: **Nedelec, Lucien, 45, boulevard de L'Ouest, F-93340 Le Raincy (FR)**
Inventeur: **Delevallee, Françoise, 48- 50, avenue de la Dame Blanche, F-94120 Fontenay Sous Bois (FR)**

(74) Mandataire: **Bourgouin, André, Département des Brevets ROUSSEL UCLAF B.P. no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 169 148 B1

LIBER, STOCKHOLM 1989

## Description

La présente invention a pour objet de nouveaux dérivés de l'indole, leur procédé de préparation, leur application comme médicament et les compositions les renfermant.

Des dérivés du pipéridin ou du 1,2,5,6-tétrahydropyridin-3yl-indole, sont décrits dans les brevets européens 0021924'0071521, 0071520 et 0112191, comme médicaments stimulants dopraminergiques.

Ces produits sont donc différents des produits de la présente demande qui sont des dérivés du pipéridin ou du 1,2,5,6-tétrahydropyridin-4yl-indole et sont doués de propriétés analgésiques.

L'invertion a pour objet les composés de formule (I):

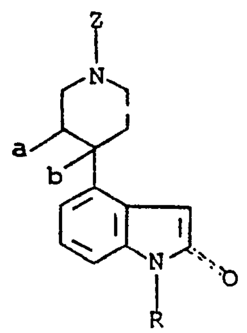

(I)

dans laquelle

R représente un atome d'hydrogène, un radical alkyle renfermant de 1 à 8 atomes de carbone ou un radical aralkyle renfermant de 7 à 12 atomes de carbone,

Z représente un atome d'hydrogène, un radical alkyle renfermant de 1 à 8 atomes de carbone, un radical alkényle renfermant de 2 à 8 atomes de carbone, un radical cyanoalkyle renfermant de 3 à 8 atomes de carbone, un radical hydroxyalkyle renfermant de 2 à 8 atomes de carbone, un radical de formule $-(CH_2)_n-O-Ar$, de formule $-(CH_2)_n-\underset{\underset{O}{\|}}{C}-Ar$ ou de formule $-CH_2-\underset{\underset{OH}{|}}{CH}-CH_2-O-Ar$, dans lesquelles n représente un nombre entier pouvant varier de 2 à 8 et Ar représente un radical aryle ou hétéroaryle, tels qu'un radical phényle, naphtyle, pyridyle, thiényl et thiazolyle éventuellement substitué par un ou plusieurs radicaux choisis dans le groupe formé par les radicaux alkyle renfermant de 1 à 5 atomes de carbone, alkoxy renfermant de 1 à 5 atomes de carbone, les atomes d'halogène, le radical hydroxy, les radicaux hydroxyalkyle renfermant de 1 à 5 atomes de carbone, les radicaux alkényle, alkényloxy, alkynyle, alkynyloxy, nitro, amino, trifluorométhyle, ou Z représente un radical aralkyle renfermant de 7 à 12 atomes de carbone éventuellement substitué par un ou plusieurs radicaux choisis dans le groupe formé par les radicaux alkyle renfermant de 1 à 5 atomes de carbone, alcoxy renfermant de 1 à 5 atomes de carbone, les atomes d'halogène, les radicaux hydroxyle, trifluorométhyle, trifluoro-méthoxy, nitro et amino ou Z représente un radical cycloalkylalkyle renfermant de 4 à 12 atomes de carbone et,

- ou bien a et b représentent chacun un atome d'hydrogène,
- ou bien a représente un atome d'hydrogène et b représente un radical hydroxyle ou un radical alkoxy renfermart de 1 à 8 atomes de carbone,
- ou bien a et b forment ensemble une double liaison carbone-carbone et le groupement 2-oxo en pointillés sur le noyau indole signifie la présence éventuelle du groupement 2-oxo, étant entendu que lorsque le groupement 2-oxo est présert sur le noyau indole, la double liaison carbone-carbone du noyau indole n'existe plus, ainsi que les sels d'addition avec les acides des composés de formule (I).

Lorsque R représente un radial alkyle, il s'agit de préférence du radical méthyle, éthyle, propyle, isopropyle, butyle, isobutyle ou n-pentyle.

Lorsque R représente un radical aralkyle, il s'agit de préférence du radical benzyle.

Lorsque Z représente un radical alkyle ou hydroxyalkyle, alkyle représente de préférence un radical méthyle, éthyle, n-propyle ou isopropyle, n-butyle ou isobutyle, n-pentyle.

Lorsque Z représente un radical cyanoalkyle, alkyle représente de préférence un radical éthyle, n-propyle ou isopropyle, n-butyle ou isobutyle, n-pentyle.

Lorsque Z représente un radical $-(CH_2)_n-O-Ar$ ou $-CH_2)_n-\underset{\underset{O}{\|}}{C}-Ar$ n est de préférence un nombre entier pouvant varier de 2 à 4.

Le radical Ar peut être substitué par un ou plusieurs radicaux alkyle, alkoxy, hydroxyalkyle, tels que ceux mentionnés ci-dessus pour R et Z, atomes d'halogène tels que chlore, brome ou fluor, alkényles tels que éthényle, propényle, alkényloxy tels que éthényloxy ou propényloxy, alkynyles tels que éthynyle ou propynyle, alkynyloxy tel que propynyloxy.

Lorsque Z représente un radical aralkyle, on entend de préférence par aralkyle un radical benzyle ou phénéthyle ou phénylpropyle.

Lorsque Z représente un radical aralkyle substitué par un ou plusieurs atomes d'halogène, on entend de préférence par halogène un atome de chlore ou de brome.

Lorsque Z représente un radical aralkyle substitué par un ou plusieurs radicaux alkyles, on entend de préférence par alkyle, un radical méthyle ou éthyle.

Lorsque Z représente un radical aralkyle substitué par un ou plusieurs radicaux alkoxy, on entend de préférence par alkoxy, un radical méthoxy ou éthoxy.

Lorsque Z représente un radical cycloalkylalkyle, il s'agit de préférence d'un radical cyclopropylalkyle ou cyclohexylalkyle, par exemple du radical cyclopropylméthyle, cyclopropyléthyle ou cyclopropyl n-propyle, cyclohexylméthyle, cyclohexyléthyle ou cyclohexyl n-propyle.

Lorsque b représente un radical alkoxy, il s'agit de préférence du radical méthoxy ou éthoxy.

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne notamment les composés de formule (I) pour lesquels R représente un atome d'hydrogène ou un radical alkyle renfermant de 1 à 4 atomes de carbone, ainsi que leurs sels d'addition avec les acides.

L'invention concerne aussi particulièrement les composés de formule (I) pour lesquels Z représente un atome d'hydrogène ou un radical alkyle renfermant de 1 à 4 atomes de carbone ou un radical $-(CH_2)_n-O-Ar$ ou $-CH_2-\underset{OH}{CH}-CH_2-O-Ar$ dans lesquels n représente un nombre entier pouvant varier de 2 à 4 et Ar un radical phényle ou thiényle éventuellement substitués, ainsi que leurs sels d'addition avec les acides et plus particulièrement parmi ceux- ci, ceux pour lesquels Z représente un radical $-CH_2-\underset{OH}{CH}-CH_2-O-Ar$, Ar étant un radical phényle substitué par un groupement propényloxy, ainsi que leurs sels d'addition avec les acides.

L'invention concerne aussi notamment les composés de formule (I) pour lesquels a et b représentent chacun un atome d'hydrogène, ainsi que leurs sels d'addition avec les acides, ceux pour lesquels a et b forment ensemble une double liaison carbone-carbone, ainsi que leurs sels d'addition avec les acides et ceux pour lesquels a représente un atome d'hydrogène et b un radical hydroxyle ou un radical méthoxy, ainsi que leurs sels d'addition avec les acides.

Parmi les produits de formule (I), on peut citer tout particulièrement

- le 1,3-dihydro 4-/1-/2-hydroxy 3-/2-(2-propényloxy) phénoxy/ propyl/ 1,2,3,6-tétrahydro 4-pyridinyl/ 2H-indol-2-one et son chlorhydrate;
- le 4-(4-méthoxy 4-pipéridinyl) 1H-indole et son fumarate;
- le 4-(1H-indol-4-yl) -//2-(2-propényloxy) phénoxy/ méthyl/ 1,2,3,6-tétrahydro 1-pyridine éthanol et son oxalate.

L'invention a également pour objet un procédé de préparation des composés de formule (I) tels que définis ci-dessus, ainsi que de leurs sels, caractérisé en ce que l'on condense la N-benzyl 4-pipéridone avec un composé de formule (II):

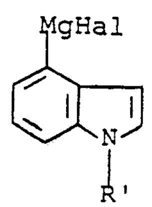

(II)

dans laquelle Hal représente un atome d'halogène et R' un radical alkyle renfermant de 1 à 8 atomes de carbone ou un radical aralkyle renfermant de 7 à 12 atomes de carbone, pour obtenir un composé de formule ($I_A$):

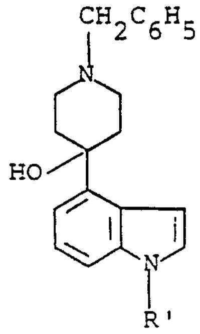

$(I_A)$

puis, si désiré, soumet le composé de formule ($I_A$) à l'action d'un agent de clivage du groupement benzyle porté par l'azote du groupement pipéridinyle, pour obtenir un composé de formule ($I_B$):

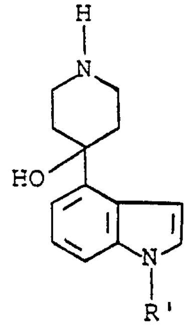

$(I_B)$

que l'on soumet, si désiré, à l'action d'un agent de déshydratation, pour obtenir un composé de formule ($I_C$):

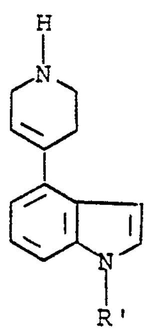

$(I_C)$

ou bien, dans le cas où R' représente un groupe benzyle, soumet le composé de formule ($I_A$), à l'action d'un agent de clivage sélectif du groupement benzyle porté par le noyau indole, pour obtenir un composé de formule ($I_D$):

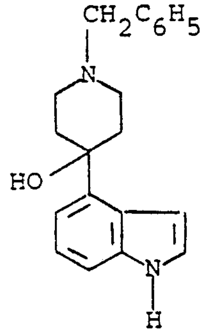

$(I_D)$

que l'on soumet, si désiré, à l'action d'un agent de clivage du groupement benzyle porté par le noyau pipéridinyle, pour obtenir un composé de formule ($I_F$):

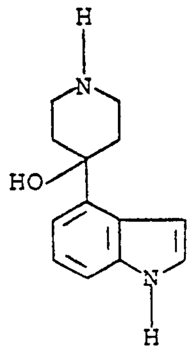

$(I_F)$

puis soumet, si désiré, chacun des composés obtenus répondant à la formule (I) dans laquelle Z représente un atome d'hydrogène, à l'action d'un agent capable d'introduire un radical Z', Z' ayant la même signification que Z, à l'exception d'hydrogène, pour obtenir les composés de formule (I) correspondants dans lesquels l'atome d'azote du noyau pipéridinyle porte un radical Z', et, si désiré, l'on soumet chacun des composés répondant à la formule (I) dans laquelle Z est différent d'un atome d'hydrogène et dans laquelle R représente un atome d'hydrogène à l'action d'un agent d'alkylation ou d'aralkylation pour obtenir les composés de formule (I) dans laquelle R représente un radical alkyle renfermant de 1 à 8 atomes de carbone ou un radical aralkyle renfermant de 7 à 12 atomes de carbone et, si désiré,

- <u>soit</u> l'on soumet l'un ou l'autre des composés obtenus répondant à la formule (I) dans laquelle b représente un radical hydroxyle à l'action d'un agent d'ethérification pour obtenir les composés de formule (I) correspondants dans laquelle b représente un radical alkoxy renfermant de 1 à 8 atomes de carbone,
- <u>soit</u> l'on soumet l'un ou l'autre des composés obtenus répondant à la formule (I) dans laquelle b représente un radical hydroxyle à l'action d'un agent de déshydratation pour obtenir les composés de formule (I) correspondants dans laquelle a et b forment ensemble une double liaison carbone-carbone,
- <u>soit</u> l'on soumet l'un ou l'autre des composés obtenus répondant à la formule (I) dans laquelle a et b forment ensemble une double liaison carbone-carbone, à l'action d'un agent de réduction, pour obtenir les composés de formule (I) correspondants dans laquelle a et b représentent chacun un atome d'hydrogène,
- <u>soit</u> l'on soumet l'un ou l'autre des composés obtenus répondant à la formule (I) dans laquelle b représente un radical hydroxyle, à l'action d'un agent de clivage du groupement hydroxyle, pour obtenir les composés de formule (I) correspondants dans laquelle a et b représentent chacun un atome d'hydrogène et, si désiré, soumet l'un ou l'autre des composés de formule (I) ainsi obtenus, à l'action d'un agent d'halogénation susceptible d'introduire un atome d'halogène en 3 du noyau indole et hydrolyse le dérivé halogéné ainsi obtenu pour préparer les composés de formule (I) comportant un groupement 2-oxo et si désiré, soumet chacun des composés de formule (I) obtenus précédemment, à l'action d'un acide pour en former le sel.

Ce procédé est notamment caractérisé en ce que l'introduction du radical Z' est réalisée:

- grâce à une réaction d'hydroxyméthylation suivie d'une réduction du méthylol intermédiaire formé, lorsque Z' est un radical méthyle,
- au moyen d'un halogénure Z'-Hal lorsque Z' est un radical alkyle renfermant de 1 à 8 atomes de carbone, un radical alkényle renfermant de 2 à 8 atomes de carbone, un radical hydroxyalkyle renfermant de 2 à 8 atomes de carbone, un radical cyanoalkyle renfermant de 3 à 8 atomes de carbone, un radical aralkyle renfermant de 7 à 12 atomes de carbone, un radical cycloalkylalkyle renfermant de 4 à 12 atomes de carbone, un groupement

$$-(CH_2)_n-O-Ar \text{ ou } -(CH_2)_n-\underset{\underset{O}{\|}}{C}-Ar,$$

- au moyen d'un époxyde de formule 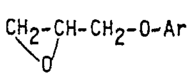 dans laquelle Ar a la signification déjà indiquée, lorsque Z' est le groupement

$$-CH_2-\overset{\overset{OH}{|}}{C}H-CH_2-O-Ar,$$

- au moyen de l'acrylonitrile lorsque Z' est un radical cyanoéthyle.

Dans un mode de réalisation préférée du procédé de l'invention:

- On utilise comme produit de départ de formule (II) un produit dans lequel Hal représente un atome de chlore ou de brome.
- L'agent de clivage du groupement benzyle porté par le noyau pipéridinyle, si a et b ne représentent pas ensemble une double liaison, est l'hydrogène en présence d'un catalyseur, par exemple, l'hydrogène en présence de palladium.
- L'agent de déshydratation est un acide fort comme l'acide chlorhydrique ou l'acide oxalique ou encore l'anhydride phosphorique.
- L'agent de clivage du groupement benzyle, porté par le noyau de l'indole est le sodium dans l'ammoniac à basse température.
- Pour obtenir à partir des composés de formule (I) dans laquelle R est un atome d'hydrogène, des composés de formule (I) dans laquelle R est un alkyle ou un aralkyle, on utilise comme agent d'alkylation ou d'aralkylation, un halogénure d'alkyle ou d'aralkyle, par exemple un chlorure, un bromure ou un iodure d'alkyle ou d'aralkyle. La réaction s'effectue en présence d'une base, par exemple la potasse dans du diméthyl sulfoxyde. On utilise avantageusement l'hydrure de sodium en présence de diméthylformamide.
- Pour obtenir, à partir des composés de formule (I) dans laquelle b est un hydroxyle, des composés de formule (I) dans laquelle b est un radical alkoxy, on utilise comme agent d'éthérification un alcool en milieu acide anhydre.
- Pour obtenir, à partir des composés de formule (I) dans laquelle b est un hydroxyle, des composés de formule (I) dans laquelle a et b représentent chacun un atome d'hydrogène, on utilise comme agent de clivage du groupement OH le lithium dans l'ammoniac liquide à basse température, par exemple de -35 à -60° C.

Pour obtenir, à partir des composés de formule (I) dans laquelle a et b forment une double liaison carbone-carbone, des composés de formule (I) dans laquelle a et b représente chacun un atome d'hydrogène, on utilise comme agent de réduction l'hydrogène en présence d'un catalyseur, par exemple le palladium.

- L'agent d'halogénation susceptible d'introduire un atome d'halogène en position 3 de l'indole, est un N-halosuccinimide, par exemple, le N-chloro ou le N-bromo succinimide.
- L'hydrolyse du dérivé chloré permettant d'obtenir un composé de formule (I) ayant un groupe 2-oxo sur l'indole, s'effectue par action d'un acide tel que l'acide chlorhydrique.
- Lorsque Z' est un radical méthyle, son introduction s'effectue par une réaction d'hydroxyméthylation en utilisant l'aldéhyde formique dans le méthanol, suivie d'une réduction du méthylol intermédiaire formé par le tétrahydroborure de sodium.
- Dans l'halogénure Z'-Hal, Hal représente un atome de chlore, de brome ou d'iode.
- Lorsque Z' est un hydroxyalkyle, le groupe hydroxyle est protégé sous forme de dérivé pyrannique. La déprotection du ou des groupements hydroxy est réalisée par action d'un acide tel que l'acide chlorhydrique ou l'acide sulfurique.
- L'introduction du groupement Z de formule $-CH_2-\underset{OH}{CH}-CH_2-O-Ar$ par l'intermédiaire d'un époxyde s'effectue au reflux d'un alcool, tel que l'éthanol ou le méthanol.

La salification des composés de formule (I) est réalisée selon les méthodes usuelles, notamment en faisant réagir les composés en question avec un acide en quantité stoéchiométrique, par exemple au sein d'un alcanol, d'un mélange d'alcanols ou d'un mélange d'alcanol et d'acétate d'éthyle.

Les composés de formule (I) tels que définis ci-dessus ainsi que leurs sels d'addition avec les acides présentent d'intéressantes propriétés pharmacologiques. Ils présentent en particulier une forte affinité pour les récepteurs opiacés, notamment pour les récepteurs $\mu$ et sont doués de propriétés analgésiques de type morphinique. Certains composés de formule (I) présentent en outre une affinité pour les récepteurs K. Ces propriétés justifient leur application en thérapeutique et l'invention a également pour objet à titre de médicaments, les produits tels que définis par la formule (I) ci-dessus, ainsi que leurs sels d'addition avec les acides pharmaceutiquement acceptables.

La présente invention a tout particulièrement pour objet, à titre de médicament

- le 1,3-dihydro 4-/1-/2-hydroxy 3-/2-(2-propényloxy) phénoxy/ propyl/ 1,2,3,6-tétrahydro 4-pyridinyl/ 2H-indol-2-one et son chlorhydrate;
- le 4-(4-méthoxy 4-pipéridinyl) 1H- indole et son fumarate;
- le 4-(1H-indol-4-yl) $\alpha$-//2-(2-propényloxy) phénoxy/ méthyl/ 1,2,3,6-tétrahydro 1-pyridine éthanol et son oxalate.

Les médicaments, objet de l'invention, peuvent être utilisés dans le traitement des douleurs intenses, en particulier rebelles aux antalgiques périphériques, par exemple au cours des processus néoplasiques, dans le traitement des pancréatites, coliques néphrétiques ou biliaires, dans le traitement des douleurs postopératoires et post-traumatiques.

L'invention s'étend aux compositions pharmaceutiques renfermant comme principe acif, les médicaments définis ci-dessus.

Ces compositions pharmaceutiques peuvent être administrées par voie buccale, rectale, par voie parentérale

ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 5 et 50 mg de principe actif par jour, par voie parentérale.

Les produits de formule (II) utilisés comme produits de départ dans le procédé de l'invention sont décrits dans le brevet français n° 2 458 549 ou préparés selon les méthodes indiquées dans ce brevet.

Les exemples suivants illustrent l'invention sans toutefois la limiter.

**Exemple 1**:

Chlorhydrate de 1-(phénylméthyl) 4-/1-(phénylméthyl) 1H-indol-4-yl/ 4-pipéridinol.

a) Formation du magnésien.

On prépare le magnésien du N-benzyl 4-chloro indole de la même manière que celle indiquée au stade A de l'exemple 1 du brevet français n° 2 458 549 à partir de 9 g de magnésium et de 24,2 g de N-benzyl 4-chloro indole.

b) Chlorhydrate de 1-(phénylméthyl) 4-/1-(phénylméthyl) 1H-indol-4-yl/ 4-pipéridinol.

On refroidit à 30°C, la solution obtenue au paragraphe a) et introduit sans dépasser 40°C, 17,7 g de N-benzyl 4-pipéridone en solution dans 40 $cm^3$ de tétrahydrofuranne. On chauffe au reflux pendant 2 heures puis refroidit à 10°C et ajoute une solution aqueuse saturée de chlorure d'ammonium. On filtre l'excès de magnésium et extrait à l'acétate d'éthyle, lave avec une solution aqueuse d'hydroxyde de sodium, sèche et élimine les solvants sous pression réduite. On dissout les 42 g d'huile brune isolée précédemment dans 400 $cm^3$ d'acétate d'éthyle et forme le chlorhydrate par addition d'une solution saturée d'acide chlorhydrique dans l'acétate d'éthyle puis filtre et sèche sous pression réduite. On recristallise le chlorhydrate ainsi obtenu dans l'isopropanol à 50 % de méthanol et obtient 17,3 g de produit pur fondant à 270°C.

c) Libération de la base.

On libère la base correspondante par traitement à la soude et obtient par extraction à l'acétate d'éthyle et évaporation du solvant, le produit recherché.

**Exemple 2**:

1-(phénylméthyl) 4-/1H-indol-4-yl/ 4-pipéridinol et chlorhydrate.

a) formation de la base.

On introduit, sous azote, sous agitation à -40°C, une solution de 6,5 g de 1-(phénylméthyl) 4-/1-(phénylméthyl) 1H-indol-4-yl/ 4-pipéridinol (obtenu au stade c de l'exemple 1) dans 250 $cm^3$ de tétrahydrofuranne, dans 500 $cm^3$ d'ammoniac liquide. On ajoute, par portions, à -40°C, 4 g de sodium, maintient 30 minutes à -40°C puis décolore la solution par addition de chlorure d'ammonium et évapore l'ammoniac. On reprend le résidu à l'eau et extrait à l'acétate d'éthyle. On lave à l'eau, sèche et chasse les solvants sous pression réduite. On chromatographie sur silice le produit obtenu (éluant: cyclohexane-chloroforme-triéthylamine (6 - 3 - 1) puis empâte au cyclohexanne, filtre, sèche et obtient 12,2 g de produit attendu fondant à 108°C.

b) Formation du chlorhydrate.

On dissout le produit obtenu au stade a) dans l'acétate d'éthyle et ajoute une solution saturée d'acide chlorhydrique dans l'acétate d'éthyle. On obtient 13,7 g de produit attendu fondant à 200°C.

**Exemple 3**:

4-(1H-indol-4-yl) 4-pipéridinol et chlorhydrate.

a) Formation du chlorhydrate.

On hydrogène à 40°C 13,7 g de chlorhydrate de 1-(phénylméthyl) 4-/1H-indol-4-yl/ 4-pipéridinol obtenu au stade b de l'exemple 2 dans 600 $cm^3$ de méthanol et en présence de 4,7 g de charbon palladié. On filtre, concentre à sec à 40°C sous pression réduite, empâte à l'éther le résidu obtenu, filtre, sèche sous pression réduite à 20 - 25°C et obtient 7,9 g de produit fondant à 270°C.

b) Base.

On libère la base correspondante en traitant le produit obtenu au stade a à la soude en solution aqueuse et obtient par extraction à l'acétate d'éthyle le produit recherché.

**Exemple 4:**

4-(1H-indol-4-yl) 1-méthyl 4-pipéridinol et fumarate.

a) Hydroxyméthylation.

On introduit lentement entre 8° C et 10° C, 2,5 $cm^3$ d'une solution aqueuse d'aldéhyde formique à 37 % dans 5 g de 4-(1H-indol-4-yl) 4-pipéridinol obtenu au stade b de l'exemple 3 dissous dans 50 $cm^3$ de méthanol. On agite 15 minutes à 5° C/10° C, observe une cristallisation dans le milieu réactionnel, dilue par 100 $cm^3$ de méthanol, laisse revenir à température ambiante, agite jusqu'à dissolution totale.

b) Réduction.

On refroidit la solution obtenue au stade a) à 5 - 8° C, ajoute sans dépasser 11° C, 2,62 g d'hydroborure de sodium à 95 %, laisse revenir à température ambiante, agite 1 heure. On verse le mélange réactionnel dans 300 $cm^3$ d'eau. On filtre le précipité obtenu, lave à l'eau, sèche sous pression réduite à 70° C et obtient 4,45 g de produit. On extrait le filtrat à l'acétate d'éthyle après alcalinisation au carbonate de potassium, lave à l'eau salée, sèche, évapore et obtient 1,27 g d'une huile. On réunit cette huile aux 4,45 g de produit obtenu précédemment, la dissout dans l'acétone, effectue une chromatographie sur silice (éluant: chloroforme-acétone-triéthylamin 6 - 3 - 1). On obtient 2,8 g de produit attendu fondant à 205 - 206° C.

c) Obtention du fumarate neutre.

A une solution de 1,41 g d'acide fumarique dans l'isopropanol, on ajoute 2,8 g de produit obtenu au stade b) précédent en solution dans 280 $cm^3$ d'isopropanol. On dissout au reflux en ajoutant de l'isopropanol, glace, cristallise, filtre, rince à l'alcool isopropylique. On obtient 0,730 g de produit attendu fondant à 240° C.

d) Obtention du fumarate acide.

On concentre les liqueurs mères, glace, filtre, rince à l'isopropanol et sèche sous pression réduite à 70° C et obtient 2,03 g de produit attendu fondant à 210 - 211° C.

**Exemple 5:**

4-(1-méthyl 1,2,3,6-tétrahydro pyridin-4-yl) 1H-indole et fumarate.

a) Formation de la base.

On chauffe au reflux pendant 1 heure 30 minutes, 6,4 g de 4-/1H-indol 4-yl/ 1-méthyl 4-pipéridinol obtenu au stade b) de l'exemple 4 et 200 $cm^3$ d'acide chlorhydrique 1N. On refroidit à la température ambiante, dilue à l'eau, alcalinise par addition de carbonate dipotassique et extrait à l'acétate d'éthyle, lave à l'eau, sèche et chasse le solvant sous pression réduite. On obtient 5,7 g de produit recherché que l'on purifie par chromatographie sur silice (éluant: cyclohexane-chloroforme-triéthylamine 6 - 3 - 1). On isole 2,5 g de produit recherché fondant à 164° C.

b) Formation du fumarate neutre.

On dissout le produit obtenu au stade a) dans 150 $cm^3$ d'isopropanol à 60° C puis ajoute 1,4 g d'acide fumarique. Il y a dissolution puis cristallisation. On effectue à nouveau la dissolution par addition de 100 $cm^3$ de méthanol, concentre, glace, filtre et sèche sous pression réduite et obtient 3,4 g de produit fondant à 170° C puis 184° C.

**Exemple 6:**

1-propyl 4-/1H-indol-4-yl/ 4-pipéridinol.

On mélange sous azote et sous agitation 6 g de chlorhydrate de 4-(1H-indol-4-yl) 4-pipéridinol obtenu au stade a) de l'exemple 3, 120 $cm^3$ de diméthylformamide, 7,5 g de carbonate de sodium et 3 $cm^3$ d'iodure de propyle. On abandonne la suspension obtenue, à température ambiante pendant 5 heures. On extrait à l'acétate d'éthyle, lave à l'eau, sèche, élimine le solvant sous pression réduite et obtient 5,15 g de produit attendu.

**Exemple 7:**

4-(1-propyl 1,2,3,6-tétrahydro pyridin-4-yl) 1H-indole et son fumarate acide.

a) Formation de la base.

On opère de la manière identique à celle de l'exemple 5, stade a) à partir de 5,15 g de 1-propyl 4-/1H-indol-4-yl/ 4-pipéridinol obtenu à l'exemple 6. On obtient 2,55 g de produit attendu fondant à 105°C.

b) Formation du fumarate acide.

On opère de manière identique à celle de l'exemple 5, stade b et obtient 3,2 g de produit attendu fondant à 192°C.

**Exemple 8:**

4-(4-pipéridinyl) 1H-indole.

On introduit, sous agitation et sous azote à -40°C, une solution de 2,7 g d'éthanol, de 11 $cm^3$ de tétrahydrofuranne et de 650 mg de 4-(1H-indol-4-yl) 4-pipéridinol préparé au stade b) de l'exemple 3, dans 60 $cm^3$ d'ammoniac. On ajoute ensuite en 1 heure, par petites fractions, 200 mg de lithium, puis évapore l'ammoniac à température ambiante, reprend le résidu à l'eau, extrait à l'acétate d'éthyle, lave à l'eau et eau salée, sèche, filtre, élimine les solvants sous pression réduite à 40°C. On obtient 0,596 g de produit brut que l'on purifie par chromatographie sur silice (éluant: chloroforme-méthanol-triéthylamine 7 - 2 - 1) et obtient 0,350 g de produit attendu fondant à 230°C.

**Exemple 9:**

4-(1H-indol-4-yl) α-//2-(2-propényloxy) phénoxy/ méthyl/ 1-pipéridine éthanol et son succinate neutre.

a) Formation de la base.

On porte au reflux pendant 2 heures, sous agitation et sous azote, la suspension contenant 3,5 g de 4-(4-pipéridinyl) 1H-indole préparé comme à l'exemple 8, 3,95 g de 2-/2-(prop-2-ényloxy) phénoxy/ méthyl oxiranne décrite dans le brevet belge n° 669 402 et 70 $cm^3$ d'éthanol. On évapore les solvants puis sépare le produit désiré par chromatographie sur silice (éluant: cyclohexane-chloroforme-triéthylamine 6 - 3 - 1) et obtient 4,88 g de produit attendu présentant un Rf de 0,13.

b) Formation du succinate neutre.

On dissout 3,25 g de base dans 150 $cm^3$ d'isopropanol et ajoute 472 mg d'acide succinique. On chauffe 30 minutes environ la solution obtenue, concentre, amorce la cristallisation, abandonne 20 heures à température ambiante, filtre, sèche sous pression réduite. On obtient 2,8 g de produit attendu fondant à 125°C.

**Exemple 10:**

4-hydroxy 4-(1H-indol-4-yl) α-//2-(2-propényloxy) phénoxy/ méthyl/ 1-pipéridine éthanol et son chlorhydrate.

a) Formation de la base.

On chauffe au reflux pendant 5 heures sous agitation et sous azote une suspension contenant 1 g de chlorhydrate de 4-(1H-indol-4-yl) 4-pipéridinol (produit de l'exemple 3, stade a), 20 $cm^3$ d'éthanol et 900 mg de 2-/2-(prop-2-ényloxy) phénoxy/ méthyl oxiranne. On dilue à l'eau, alcalinise par addition de soude, extrait à l'acétate d'éthyle et évapore à sec. On sépare le produit désiré par chromatographie sur silice (éluant: chloroforme-acétone, triéthylamine 8 - 1 - 1) et obtient 710 mg de produit attendu présentant un Rf de 0,25.

b) Formation du chlorhydrate.

On dissout 1,6 g de base dans 200 $cm^3$ d'acétate d'éthyle et 20 $cm^3$ de méthanol, ajoute une solution saturée d'acide chlorhydrique dans l'acétate d'éthyle. On amorce la cristallisation, concentre, glace, filtre, sèche sous pression réduite et obtient 1,6 g de produit fondant à 185°C.

**Exemple 11:**

4-(1H-indol-4-yl) α-//2-(2-propényloxy) phénoxy/ méthyl/ 1,2,3,6-tétrahydro 1-pyridine éthanol et son oxalate neutre.

a) Formation de la base.

On chauffe au reflux pendant 3 heures une solution contenant 9,3 g de 4-hydroxy 4-(1H-indol-4-yl) α-//2-(2-propényloxy) phénoxy/ méthyl/ 1-pipéridinol préparé au stade a) de l'exemple 10, 300 $cm^3$ d'acide chlorhydrique 1N et 100 $cm^3$ d'éthanol. On dilue à l'eau, alcalinise, sature la phase aqueuse par du carbonate dipotassique et extrait à l'acétate d'éthyle. On sépare le produit désiré par chromatograhie sur silice (éluant: cyclohexane-chloroforme-triéthylamine 6 - 3 - 1) et obtient 7,7 g de produit attendu présentant un Rf de 0,05.

b) Formation de l'oxalate neutre.

On dissout 7,7 g de base dans 300 $cm^3$ d'ispropanol et 300 $cm^3$ de méthanol et ajoute 1,2 g d'acide oxalique.

On chauffe au reflux pendant 30 minutes environ, concentre, amorce la cristallisation, glace, filtre, sèche sous pression réduite. On obtient 6,7 g de produit attendu fondant à 163°C.

**Exemple 12**:

4-(1-méthyl 1H-indol-4-yl) α-//2-(2-propényloxy) phénoxy/ méthyl/ 1,2,3,6-tétrahydro 1-pyridine éthanol et son sulfate acide.

a) Formation de la base.

On chauffe pendant 30 minutes à 50°C, sous agitation et sous azote, 6,1 g de 4-(1H-indol4-yl) α-//2-(2-propényloxy) phénoxy/ méthyl/ 1,2,3,6-tétrahydro 1-pyridine éthanol préparé au stade a) de l'exemple 11, 50 $cm^3$ de diméthylformamide, 725 mg d'hydrure de sodium à 55 %. On refroidit ensuite à 0°C et introduit 1 $cm^3$ d'iodure de méthyle, chauffe 3 heures à 40°C, refroidit à 10°C et introduit 100 $cm^3$ d'eau puis extrait à l'acétate d'éthyle. On sépare le produit désiré par chromatographie sur silice (éluant: cyclohexane-chloroforme-triéthylamine 6 - 3 - 1). On obtient 3,2 g de produit attendu présentant un Rf de 0,20.

b) Formation du sulfate acide.

On dissout 2,9 g de base dans 100 $cm^3$ d'isopropanol et ajoute une solution molaire d'acide sulfurique dans l'isopropanol jusqu'à pH acide. On concentre jusqu'à 50 $cm^3$, glace, filtre et sèche sous pression réduite. On obtient 3,1 g de produit attendu fondant à 150°C.

**Exemple 13**:

1,3-dihydro 4-/1-/2-hydroxy 3-/2-(2-propényloxy) phénoxy/ propyl/ 1,2,3,6-tétrahydro 4-pyridinyl/ 2H-indol-2-one et son chlorhydrate.

a) Formation de la base.

- Formation du dérivé chloré intermédiaire.

On laisse pendant 1 heure, sous agitation et sous azote, une solution contenant 6,6 g de 4-(1H-indol-4-yl) α-//2-(2-propényloxy) phénoxy/ méthyl/ 1,2,3,6-tétrahydro 1-pyridinéthanol préparé à l'exemple 11 stade a), 80 $cm^3$ d'acide acétique et 2,5 g de N-chloro succinimide. On dilue à l'eau, alcalinise par addition de carbonate dipotassique, extrait à l'acétate d'éthyle et obtient 8,8 g de dérivé chloré que l'on utilise tel quel au stade suivant.

- Hydrolyse du dérivé chloré.

On laisse 20 heurs à température ambiante, sous agitation et sous azote, 8,8 g de dérivé chloré obtenu précédemment dans 100 $cm^3$ d'éthanol et 200 $cm^3$ d'acide chlorhydrique N. On alcalinise par addition de soude, sature la phase aqueuse par du carbonate dipotassique et extrait à l'acétate d'éthyle. On obtient 6,7 g d'une résine que l'on dissout dans 200 $cm^3$ d'acétate d'éthyle au reflux, concentre, amorce la cristallisation glace, filtre, sèche sous pression réduite et obtient 3,1 g de produit attendu fondant à 130 - 135°C.

b) Formation du chlorhydrate.

On dissout 4,1 g de base obtenue comme précédemment dans 400 $cm^3$ d'isopropanol et 200 $cm^3$ de méthanol au reflux. On refroidit, ajoute une solution saturée d'acide chlorhydrique dans l'isopropanol jusqu'à pH acide. On élimine le méthanol, concentre, glace, filtre et sèche sous pression réduite à 80°C. On obtient 3,9 g de produit brut que l'on recristallise dans l'acétate d'éthyle et le méthanol au reflux. On concentre, glace, filtre, sèche sous pression réduite à 80°C et obtient 3,6 g de produit attendu fondant à 190°C.

**Exemple 14**:

4-(4-méthoxy 4-pipéridinyl) 1H-indole et son fumarate acide.

a) Formation de la base.

On laisse pendant 1 heure, à température ambiante, sous agitation et sous azote, 13 g de chlorhydrate de 4-(1H-indol-4-yl) 4-pipéridinol préparé au stade a) de l'exemple 3 dans 140 $cm^3$ de méthanol et 70 $cm^3$ d'une solution saturée d'acide chlorhydrique dans le méthanol fraîchement préparée. On alcalinise par addition de carbonate dipotassique à 0/5°C, dilue dans l'eau, extrait à l'acétate d'éthyle, sèche, filtre, élimine les solvants sous pression réduite. On isole le produit désiré par chromatographie sur silice (éluant: chloroforme-acétone-triéthylamine 6 - 3 - 1) et obtient 8,8 g de produit brut que l'on purifie par cristallisation. On dissout 3,4 g de produit dans l'acétone au reflux, filtre à chaud, concentre, glace, amorce la cristallisation, filtre et sèche sous pression réduite à température ambiante. On obtient 2,45 g de produit attendu fondant à 216°C.

b) Formation du fumarate acide.

On dissout 2,45 g de base dans 200 $cm^3$ d'isopropanol et ajoute 1,23 g d'acide fumarique, chauffe 30 minutes au reflux, concentre, glace, filtre, sèche sous pression réduite à 80°C et obtient 3,15 g de produit fondant à 220°C.

**Exemple 15:**

4-(1H-indol-4-yl) 4-méthoxy α-//2-(2-propényloxy) phénoxy/ méthyl/ 1-pipéridine éthanol et son tartrate neutre.
a) Formation de la base.
On chauffe pendant 4 heures au reflux, sous agitation et sous azote, 3,2 g de 4-(4-méthoxy 4-pipéridinyl) 1H-indole préparé au stade a) de l'exemple 14, 3,15 g de 2-//2-(prop-2-ényloxy) phénoxy/ méthyl/ oxiranne et 100 $cm^3$ d'éthanol. On élimine l'éthanol à 50°C sous pression réduite et sépare le produit désiré par chromatographie sur silice (éluant: chloroforme-cyclohexanne-triéthylamine 6 - 3 - 1). On obtient 3,1 g de produit attendu présentant un Rf de 0,60.
b) Formation du tartrate neutre.
On dissout 2,95 g de base dans l'isopropanol et ajoute 1 g d'acide DL tartrique, chauffe au reflux 15 minutes, concentre, glace, filtre et sèche sous pression réduite à 80°C. On obtient 2,6 g de produit attendu fondant à 178°C.

**Exemple 16:**

1-butyl 4-(1H-indol-4-yl) 4-pipéridinole.
On opère comme à l'exemple 6 à partir de 6,3 g de chlorhydrate de 4-(1H-indol-4-yl) 4-pipéridinole obtenu au stade a) de l'exemple 3, 125 $cm^3$ de diméthylformamide, 5,36 $cm^3$ de 1-bromobutane et 6,625 g de carbonate de sodium. Après recristallisation dans le méthanol du produit brut obtenu, on recueille 5,33 g de produit attendu. F $\simeq$ 100°C.

**Exemple 17:**

4-(1-butyl-1,2,3,6-tétrahydropyridin-4-yl) 1H-indole et son fumarate acide.
a) Formation de la base.
On chauffe au reflux pendant 3 heures 30 minutes, 5 g de produit obtenu à l'exemple 16 en solution dans 80 $cm^3$ d'éthanol et 160 $cm^3$ d'acide chlorhydrique 1N. On refroidit, alcalinise à l'aide de soude 2N, extrait à l'acétate d'éthyle, lave à l'eau, sèche et chasse le solvant sous pression réduite. On obtient 5,3 g de produit brut que l'on purifie par chromatographie sur silice (éluant: chloroforme-cyclohexane-triéthylamine 6 - 3 - 1) et recueille 3,59 g de produit attendu. F = 91°C.
b) Formation du fumarate acide.
On ajoute à température ambiante 685 mg d'acide fumarique dans une solution de 1,5 g de base ci-dessus dans 30 $cm^3$ d'éthanol. Après 1 heure 30 minutes d'agitation, on filtre le précipité, le rince à l'éthanol, le sèche à 80°C sous pression réduite et recueille 1,51 g de produit attendu. F = 181 - 182°C.

**Exemple 18:**

1,3-dihydro 4-(1-butyl-1,2,3,6-tétrahydropyridin-4-yl) 2H-indol-2-one et son chlorhydrate.
Stade A: Formation du dérivé chloré.
On opère comme à l'exemple 13 à partir de 2,1 g de la base de produit préparé à l'exemple 17, 21 $cm^3$ d'acide acétique et 1,2 g de N-chlorosuccinimide et obtient 1,75 g de dérivé chloré. F = 163 - 164°C.
Stade B: 1,3-dihydro 4-(1-butyl-1,2,3,6-tétrahydropyridin-4-yl) 2H-indol-2-one et son chlorhydrate.
a) Formation de la base.
On laisse 24 heures sous agitation et sous atmosphère inerte, à température ambiante, 1,61 g du dérivé chloré préparé au stade A en solution dans 24 $cm^3$ d'éthanol et 24 $cm^3$ d'acide chlorhydrique N. On verse le mélange réactionnel dans l'eau, alcalinise à la soude, extrait à l'acétate d'éthyle, lave à l'eau, sèche et évapore le solvant. On obtient 1,52 g de base attendue; F = 100 - 111°C.
b) Formation du chlorhydrate.
On dissout la base dans 50 $cm^3$ d'éthanol et acidifie avec une solution saturée d'acide chlorhydrique dans l'acétate d'éthyle. On laisse 16 heures à 4°C, filtre le précipité, rince à l'éthanol et sèche à 80°C sous pression réduite. Après recristallisation dans le mélange éthanol-éthanol, on recueille 0,866 g de produit attendu. F = 266°C.

**Exemple 19**:

1-éthyl 4-(1H-indol-4-yl) 4-pipéridinol.

On opère comme à l'exemple 6 à partir de 6,3 g de chlorhydrate de 4-(1H-indol-4-yl) 4-pipéridinol préparé comme au stade a de l'exemple 3, 125 $cm^3$ de diméthylformamide, 2,8 $cm^3$ de bromoéthane et 6,625 g de carbonate de potassium et obtient après extraction à l'éther et cristallisation dans le méthanol 2,11 g de produit attendu. F = 135°C.

**Exemple 20**:

4-(1-éthyl-1,2,3,6-tétrahydropyridin-4-yl) 1H-indole et son fumarate acide.

a) Formation de la base.

On opère comme au stade a) de l'exemple 17 à partir de 2 g du produit obtenu à l'exemple 19, 34 $cm^3$ d'éthanol, 66 $cm^3$ d'acide chlorhydrique N. On obtient 1,28 g de produit attendu. F = 151°C.

b) Formation du fumarate acide.

On opère comme au stade b) de l'exemple 17 à partir de 1,28 g de la base préparée ci-dessus et obtient 1,504 g de produit attendu. F = 200°C.

**Exemple 21**:

1,3-dihydro 4-(1-éthyl-1,2,3,6-tétrahydropyridin-4-yl) 2H-indol-2-one et son chlorhydrate.

Stade A: Formation du dérivé chloré.

On opère comme à l'exemple 13 stade a) à partir de 1,25 g de 4-(1-éthyl-1,2,3,6-tétrahydropyridin-4-yl) 1H-indole préparé comme au stade a) de l'exemple 20, 12 $cm^3$ d'acide acétique et 0,911 g de N-chlorosuccinimide et obtient 1,21 g de dérivé chloré.

Stade B: Chlorhydrate de 1,3-dihydro 4-(1-éthyl-1,2,3,6-tétrahydropyridin-4-yl) 2H-indol-2-one.

On agite 23 heures à température ambiante et sous atmosphère inerte 1,21 g du dérivé chloré du stade A dans 18 $cm^3$ d'éthanol et 18 $cm^3$ d'acide chlorhydrique N. Après dissolution totale, il se forme progressivement un précipité. On glace, essore le produit cristallisé, le rince à l'éthanol et le sèche. Après recristallisation dans un mélange d'éthanol et de méthanol, on recueille 0,85 g de produit attendu. F > 280°C.

**Exemple 22**:

1,3-dihydro-4-(1-propyl-1,2,3,6-tétrahydropyridin-4-yl) 2H-indol-2-one et son chlorhydrate.

a) Formation du dérivé chloré.

On opère comme à l'exemple 13 stade a) à partir de 0,689 g de 4-(1-propyl 1,2,3,6-tétrahydropyridin-4-yl) 1H-indole préparé comme à l'exemple 7 stade a), 7 $cm^3$ d'acide acétique, 0,421 g de N-chlorosuccinimide et obtient 0,581 g de dérivé chloré attendu.

b) Chlorhydrate de 1,3-dihydro-4-(1-propyl-1,2,3,6-tétrahydropyridin-4-yl) 2H-indol-2-one.

On opère comme au stade B de l'exemple 21 à partir de 0,581 g du dérivé chloré obtenu au stade a) précédent et obtient 0,369 g de produit attendu. F $\simeq$ 280°C.

**Exemple 23**:

Chlorhydrate de 1,3-dihydro 4-(1-propyl-pipéridin-4-yl) 2H-indol-2-one.

On hydrogène 1,69 g de chlorhydrate de 1,3-dihydro 4-(1-propyl-pipéridin-4-yl) 2H-indol-2-one obtenu au stade b) de l'exemple 22 dans 300 $cm^3$ de méthanol en présence de 0,8 g de charbon actif à 10 % de palladium. On filtre, évapore le solvant, recristallise le produit brut obtenu dans l'éthanol et recueille 1,24 g de produit attendu. F = 270 - 275°C.

**Exemple 24**:

4-(1H-indol-4-yl) 1-(2-phényléthyl) 4-pipéridinol et son tartrate acide.

a) Préparation de la base.

On opère comme à l'exemple 6 à partir de 8 g de chlorhydrate de 4-(1H-indol-4-yl) 4-pipéridinol obtenu au

stade a) de l'exemple 3, 100 $cm^3$ de diméthylformamide, 10 g de carbonate de sodium et 5,2 $cm^3$ de bromure de β-phényléthyle. On obtient 7,8 g de produit attendu. F = 194°C.

b) Préparation du tartrate acide.

A une solution de 1 g de base obtenue précédemment dans 200 $cm^3$ d'isopropanol, on ajoute 0,47 g d'acide DL-tartrique et chauffe 15 minutes au reflux. On concentre, glace, filtre et sèche sous pression réduite à 80°C et obtient après recristallisation dans un mélange d'isopropanol et de méthanol 1,2 g de produit attendu. F = 245°C.

**Exemple 25**:

4-/1-(2-phényléthyl) 1,2,3,6-tétrahydro 4-pyridinyl/ 1H-indole et son chlorhydrate.

a) Formation de la base.

On opère comme au stade a) de l'exemple 17 à partir de 6,4 g de la base obtenue à l'exemple 24 stade a), et obtient 6,6 g de produit brut attendu que l'on purifie par chromatographie sur silice (éluant: cyclohexane-chloroforme-triéthyl-amine 6 - 3 - 1).

b) Formation du chlorhydrate.

On dissout 4,5 g de base obtenue au stade a) précédent dans 100 $cm^3$ d'isopropanol, refroidit à 0°/5°C et ajoute une solution d'acétate d'éthyle saturée d'acide chlorhydrique jusqu'à pH acide. On filtre, lave à l'isopropanol et sèche sous pression réduite à 60°C le produit brut que l'on recristallise dans un mélange d'isopropanol et d'éthanol. On recueille 3,35 g de produit attendu. F = 230°C.

**Exemple 26**:

1,3-dihydro 4-/1-(2-phényléthyl) 1,2,3,6-tétradhydro-4-pyridinyl/ 2H-indol-2-one et son chlorhydrate.

a) Formation du dérivé chloré.

On opère comme au stade a) de l'exemple 13 à partir de 1,43 g de 4-/1-(2-phényléthyl) 1,2,3,6-tétrahydro-4-pyridinyl/ 1H-indole préparé au stade a) de l'exemple 25, 15 $cm^3$ d'acide acétique et 0,7 g de N-chlorosuccinimide. On obtient 1,8 g de produit brut que l'on purifie par chromatographie sur silice (éluant: cyclohexane-chloroforme-triéthylamine 6 - 3 - 1).

b) Chlorhydrate du 1,3-dihydro 4-/1-(2-phényléthyl) 1,2,3,6-tétrahydro-4-pyridinyl/ 2H-indol-2-one.

On chauffe 1 heure à 80°C 1 g du dérivé chloré obtenu au stade a) précédent en solution dans 30 $cm^3$ d'éthanol et 20 $cm^3$ d'acide chlorhydrique N. On laisse refroidir le milieu réactionnel, glace, filtre le produit cristallisé, le lave à l'éthanol, le sèche sous pression réduite à 80°C et obtient 0,7 g de produit attendu. F = 270°C.

**Exemple 27**:

4-(1H-indol-4-yl) 1-(2-phénoxyéthyl) 4-pipéridinol.

On opère comme à l'exemple 6 à partir de 8 g de chlorhydrate de 4-(1H-indol-4-yl) 4-pipéridinol préparé comme à l'exemple 3 stade a), 100 $cm^3$ de diméthylformamide, 10 g de carbonate de sodium et 7,6 g de β-bromophénétol. On obtient 10,2 g de produit brut que l'on recristallise dans l'isopropanol puis dans l'acétonitrile. F = 168°C.

**Exemple 28**:

4-/1-(2-phénoxyéthyl) 1,2,3,6-tétrahydro-4-pyridinyl/ 1H-indole.

On opère comme au stade a) de l'exemple 17 à partir de 7,7 g de 4-(1H-indol-4-yl) 1-(2-phénoxyéthyl) 4-pipéridinol préparé comme à l'exemple 27. Le produit brut obtenu est purifié par chromatographie sur silice (éluant : cyclohexane-chloroforme-acétate d'éthyle 6 - 3 - 1). Après cristallisation dans l'éther de pétrole (Eb: 60° - 80°C), on recueille 5,52 g de produit attendu que l'on recristallise dans l'éther isopropylique. F = 95°C.

**Exemple 29**:

1,3-dihydro 4-/1-(2-phénoxyéthyl) 1,2,3,6-tétrahydro-4-pyridinyl/ 2H-indol-2-one et son chlorhydrate.

a) Formation du dérivé chloré.

On opère comme à l'exemple 13 stade a) à partir de 3,4 g du produit obtenu à l'exemple 28, 35 $cm^3$ d'acide acétique et 1,57 g de N-chlorosuccinimide. Après purification par chromatographie sur silice (éluant: cyclohexane-chloroforme-triéthylamine 6 - 3 - 1), on obtient 3,6 g de produit attendu.

b) Chlorhydrate de 1,3-dihydro 4-/1-(2-phénoxyéthyl) 1,2,3,6-tétrahydro-4-pyridinyl/ 2H-indol-2-one.

On opère comme au stade B de l'exemple 21 à partir de 3,6 g du dérivé chloré obtenu au stade a) précédent et obtient 2,9 g de produit brut que l'on recristallise dans un mélange d'isopropanol et de méthanol. F = 270 C.

**Exemple 30**:

1-/4-(4-fluorophényl 4-oxo butyl)/4-(1H-indol-4-yl) 4-pipéridinol et son fumarate acide.

a) Formation de la base.

On mélange sous atmosphère d'azote 3 g de chlorhydrate de 4-(1H-indol-4-yl) 4-pipéridinol préparé comme au stade a) de l'exemple 3, 60 $cm^3$ de méthyl isobutylcétone, 3,8 g de carbonate de sodium et 2,4 $cm^3$ de chlorure de parafluorobutyrophénone et chauffe 24 heures au reflux. On refroidit, lave à l'acétone le milieu réactionnel, chasse les solvants à 50°C sous pression réduite. On purifie par chromatographie le produit brut (éluant: chloroforme-acétone-triéthylamine 6 - 3 - 1), le recristallise dans l'acétate d'éthyle et obtient 3,2 g de produit attendu. F = 190°C.

b) Formation du fumarate acide.

On opère comme au stade b) de l'exemple 5 à partir de 3,2 g du produit obtenu au stade a) précédent dans 300 $cm^3$ d'isopropanol au reflux et de 1 g d'acide fumarique. On obtient 3,7 g de produit attendu. F = 223°C.

**Exemple 31**:

4-/1-/(4-fluorophényl) 4-oxobutyl/ 1,2,3,6-tétrahydro 4-pyridinyl/ 1H-indole.

On opère comme à l'exemple 11 stade a) à partir de 1,4 g du produit préparé à l'exemple 30, 20 $cm^3$ d'éthanol et 40 $cm^3$ d'acide chlorhydrique N. On obtient 0,95 g de produit attendu. F = 155°C.

**Exemple 32**:

1,3-dihydro 4-/1-/(4-fluorophényl) 4-oxobutyl/ 1,2,3,6-tétrahydropyridin-4-yl/ 2H-indol-2-one et son chlorhydrate.

a) Formation du dérivé chloré.

On opère comme à l'exemple 13 stade a) à partir de 0,95 g du produit préparé à l'exemple 31, 10 $cm^3$ d'acide acétique et 0,385 g de N-chlorosuccinimide. On obtient 1 g de produit brut que l'on chromatographie sur silice (éluant: cyclohexane-chloroforme-triéthylamine 6 - 3 - 1) et recueille 0,9 g de produit attendu.

b) Chlorhydrate du 1,3-dihydro 4-/1-/(4-fluorophényl) 4-oxobutyl/ 1,2,3,6-tétrahydropyridin-4-yl/ 2H-indol-2-one.

On opère comme au stade B de l'exemple 21 à partir de 0,9 g du dérivé chloré obtenu au stade a) précédent et obtient 0,74 g de produit brut que l'on recristallise dans un mélange de méthanol et d'isopropanol. On recueille 0,65 g de produit attendu. F = 254°C.

**Exemple 33**:

1-propionitrile 4-(1H-indol-4-yl) 4-pipéridinol.

On refroidit à +5°C le mélange constitué de 2,95 g de 4-(1H-indol-4-yl) 4-pipéridinol préparé comme au stade b de l'exemple 3, dans 15 $cm^3$ d'acrylonitrile en présence d'une trace d'éther monoéthylique de l'hydroquinone, puis maintient 1 heure sous agitation. On filtre le produit cristallisé formé, le rince à l'éther puis à l'isopropanol et recueille 2,26 g de produit attendu. F = 190 - 191°C.

**Exemple 34**:

4-(1-propionitrile 1,2,3,6-tétrahydropyridin-4-yl) 1N-indole et son fumarate neutre.

a) Formation de la base.

On opère comme au stade a) de l'exemple 17 en chauffant au reflux pendant 5 heures 30 minutes 3,8 g du produit préparé comme à l'exemple 33. On obtient 2,26 g de produit attendu. F = 134 - 135°C.

b) Fumarate neutre de 4-(1-propionitrile 1,2,3,6-tétrahydropyridin-4-yl) 1H-indole.

On dissout partiellement 1,165 g de la base obtenue au stade a) ci-dessus dans 30 $cm^3$ d'éthanol, ajoute 538 mg d'acide fumarique puis 58 $cm^3$ d'éthanol et 29 $cm^3$ de méthanol et porte au reflux jusqu'à complète dissolution. On filtre à chaud, concentre partiellement, refroidit et abandonne 1 heure 30 minutes à +4° C. On filtre le produit cristallisé, rince à l'éthanol, sèche à 100° C sous pression réduite et obtient 0,872 g de produit attendu. F = 190 - 191° C.

**Exemple 35**:

Le procédé selon l'invention permet en outre de préparer le produit suivant:
-4-(1-allyl 1,2,3,6-tétrahydropyridin-4-yl) 1H-indole et son fumarate. F = 196° C.

**Exemple 36**:

On a préparé un soluté injectable (voie intra-musculaire) répondant à la formule suivante:
- Produit de l'exemple 13 ........................................ 20 mg
- Solvant stérile q.s.p. ........................................ 2 ml.

**Exemple 37**:

On a préparé des comprimés répondant à la formule suivante:
- Produit de l'exemple 13 ........................................ 20 mg
- Excipient q.s.p. ................................................ 350 mg.

(détail de l'excipient: lactose, talc, amidon, stéarate de magnésium).

**Etude Pharmacologique**

1) **Liaison au récepteur opiacé μ in vitro**.
On utilise des culots membranaires conservés à -30° C pendant environ 30 jours et préparés à partir de cerveaux de rats.

Ces culots sont remis en suspension dans le tampon Tris pH 7,7. On répartit des fractions de 2 ml dans des tubes à hémolyse et ajoute de l'$^3H$ dihydromorphine 0,7nM et du produit à étudier (Les produits sont d'abord testés à 5 · $10^{-6}$M (en triple). Lorsque les produits testés déplacent de plus de 50 % la radioactivité liée spécifiquement au récepteur, ils sont testés à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de morphine à $10^{-5}$M (en triple). On incube à 25° C pendant 40 minutes, remet au bain-marie à 0° C, 5 minutes, filtre sous vide, rince au tampon Tris pH 7,7 et compte la radioactivité en présence de scintillant Trition.

Les résultats sont exprimés:

soit directement en concentration inhibitrice 50 % ($CI_{50}$), (c'est-à-dire en concentration de produit étudié, exprimée en nM, nécessaire pour déplacer 50 % de la radioactivité spécifique fixée sur le récepteur étudié,
- soit en affinité relative de liaison (= ARL) en prenant comme référence la morphine = 100

$$ARL = \frac{CI_{50} \text{ morphine x 100}}{CI_{50} \text{ de produit étuidé}}$$

Résultats:

| | $CI_{50}$ | ARL |
|---|---|---|
| Exemple 11 | 9,43 | 22,43 |
| Exemple 13 | 1,4 | 270 |
| Exemple 14 | 28 | 13 |

| | | |
|---|---|---|
| Exemple 26 | 59 | 8,2 |
| Exemple 29 | 11 | 23 |
| Exemple 32 | 3,5 | 62 |

2) **Liaison au récepteur opiacé K in vitro.**

On utilise des culots membranaires conservés à -30°C pendant environ 30 jours et préparés à partir de cervelets de cobayes.

Ces culots sont remis en suspension dans le tampon Tris pH 7,7. On répartit des fractions de 2 ml dans des tubes à hémolyse et ajoute de la $9^3H$ Ethylkétocyclazocine 1nM et le produit à étudier. (Les produits sont d'abord testés à $5 \cdot 10^{-6}M$ (en triple). Lorsque les produits testés déplacent de plus de 50 % la radioactivité liée spécifiquement au récepteur, ils sont testés à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de produit connu sous le nom U 50488 H (réf. Up. John) à $10^{-5}M$ (en triple). On incube à 25°C pendant 40 minutes, remet au bain-marie à 0°C, 5 minutes, filtre sous vide, rince au tampon Tris pH 7,7 et compte la radioactivité en présence de scintillant Trition.

Les résultats sont exprimés:

- soit directement en concentration inhibitrice 50 % ($CI_{50}$), (c'est-à-dire en concentration de produit étudié, exprimée en nM, nécessaire pour déplacer 50 % de la radioactivité spécifique fixée sur le récepteur étudié,
- soit en affinité relative de liaison (= ARL) en prenant comme référence le produit U 50 488 H

$$ARL = \frac{CI_{50}\ (U\ 50488\ H) \times 100}{CI_{50}\ \text{de produit étuidé}}$$

Résultats:

| | $CI_{50}$ | ARL |
|---|---|---|
| Exemple 7 | 182 | 1,4 |
| Exemple 32 | 460 | 1,3 |
| Exemple 29 | 152 | 1,1 |

3) **Activité analgésique**

- Test de la plaque chaude.

Des souris femelles, pesant 22 à 24 g, sont placées une par une sur une plaque de cuivre maintenue à 56°C: la réaction à la douleur se manifeste par le léchage des pattes avant de l'animal; le temps de cette réaction est noté et l'on ne retient que les souris réagissant en moins de 8 secondes.

Les animaux sont répartis par groupes homogènes et traités par les produits à étudier, un groupe ne recevant que le véhicule. Le temps de réaction à la douleur est de nouveau mesuré 30 à 60 minutes après le traitement. La dose active ou $DA_{100}$ est la dose qui augmente le temps de réaction de 100 %, 60 minutes après le traitement compte tenu des variations du temps de réaction des animaux témoins.

| | $DA_{100}$ |
|---|---|
| Exemple 14 | 0,5 mg/kg<br>voie intracérébro ventriculaire |
| Exemple 13 | 20 mg/kg<br>voie orale |

L'activité analgésique de ces produits est antagonisée par la naloxone.

- Bibliographie.

EDDY, N.B and LEIMBACH, D. "Synthetic analgesics, II Diethienylbutenyl and diethienylbutylamine." J. Pharmacol. Exp. Thev., 1953, 107, 385.

**Revendications**

1. Les composés de formule (I):

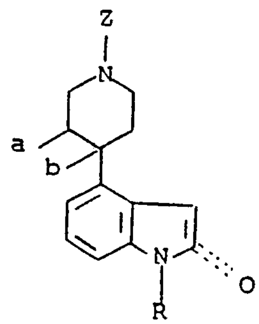

(I)

dans laquelle
R représente un atome d'hydrogène, un radical alkyle renfermart de 1 à 8 atomes de carbone ou un radical aralkyle renfermart de 7 à 12 atomes de carbone,
Z représente un atome d'hydrogène, un radical alkyle renfermant de 1 à 8 atomes de carbone, un radical alkényle renfermant de 2 à 8 atomes de carbone, un radical cyanoalkyle renfermant de 3 à 8 atomes de carbone, un radical hydroxyalkyle renfermant de 2 à 8 atomes de carbone, un radical de formule $-(CH_2)_n-O-Ar$, de formule $-(CH_2)_n-\underset{\underset{O}{\|}}{C}-Ar$, ou de formule $-CH_2-\underset{\underset{OH}{|}}{CH}-CH_2-O-Ar$, dans lesquelles n représente un nombre entier pouvant varier de 2 à 8 et Ar représente un radical phényle, naphtyle, pyridyle, thiényle, thiazolyle éventuellement substitué par un ou plusieurs radicaux choisis dans le groupe formé par les radicaux alkyle renfermant de 1 à 5 atomes de carbone, alkoxy renfermant de 1 à 5 atomes de carbone, les atomes d'halogène, le radical hydroxy, les radicaux hydroxyalkyle renfermart de 1 à 5 atomes de carbone, les radicaux alkényle, alkényloxy, alkynyle, alkynyloxy, nitro, amino, trifluorométhyle, ou Z représente un radical aralkyle renfermant de 7 à 12 atomes de carbone éventuellement substitué par un ou plusieurs radicaux choisis dans le groupe formé par les radicaux alkyle renfermant de 1 à 5 atomes de carbone, alcoxy renfermant de 1 à 5 atomes de carbone, les atomes d'halogène, les radicaux hydroxyle, trifluorométhyle, trifluorométhoxy, nitro et amino ou Z représente un radical cyclo-alkylalkyle renfermant de 4 à 12 atomes de carbone et,
- ou bien a et b représentent chacun un atome d'hydrogène,
- ou bien a représente un atome d'hydrogène et b représente un radical hydroxyle ou un radical alkoxy renfermant de 1 à 8 atomes de carbone,
- ou bien a et b forment ensemble une double liaison carbone-carbone et le groupement 2-oxo en pointillés sur le noyau indole signifie la présence éventuelle du groupement 2-oxo, étant entendu que lorsque le groupement 2-oxo est présent sur le noyau indole, la double liaison carbone-carbone du noyau indole n'existe plus, ainsi que les sels d'addition avec les acides des composés de formule (I).

2. Les composés de formule (I) tels que définis à la revendication 1, pour lesquels R représente un atome d'hydrogène ou un radical alkyle renfermant de 1 à 4 atomes de carbone, ainsi que leurs sels d'addition avec les acides.

3. Les composés de formule (I), tels que définie aux revendications 1 et 2, pour lesquels Z représente un atome d'hydrogène ou un radical alkyle renfermant de 1 à 4 atomes de carbone ou un radical $-(CH_2)_n-O-Ar$ ou $-CH_2-\underset{\underset{OH}{|}}{CH}-CH_2-O-Ar$ dans lesquels n représente un nombre entier pouvant varier de 2 à 4 et Ar un radical phényle ou thiényle éventuellement substitués, ainsi que leurs sels d'addition avec les acides.

4. Les composés de formule (I), tels que définis aux revendications 1 à 3, pour lesquels Z représente un radical $-CH_2-\underset{\underset{OH}{|}}{CH}-CH_2-O-Ar$, Ar étant un radical phényle substitué par un groupement propényloxy, ainsi que leurs sels d'adddition avec les acides.

5. Les composés de formule (I), tels que définis aux revendications 1 à 4, pourlesquels a et b représentent chacun un atome d'hydrogène, ainsi que leurs sels d'addition avec les acides.

6. Les composés de formule (I), tels que définis aux revendications 1 à 4, pour lesquels a et b forment ensemble une double liason carbone-carbone, ainsi que leurs sels d'addition avec les acides.

7. Les composés de formule (I), tels que définis à l'une quelconque des revendications 1 à 4, pour lesquels a représente un atome d'hydrogène et b un radical hydroxyle ou un radical méthoxy, ainsi que leurs sels d'addition avec les acides.

8. L'un quelconque des composés de formule (I) dont les noms suivent:
-le 1,3-dihydro 4-/1-/2-hydroxy 3-/2-(2-propényloxy) phénoxy/ propyl/ 1,2,3,6-tétrahydro 4-pyridinyl/ 2H-indol-2-one et son chlorhydrate;
- le 4-(4-méthoxy 4-pipéridinyl) 1H-indole et son fumarate;
- le 4-(1H-indol-4-yl) α-//2-(2-propényloxy/ méthyl/ 1,2,3,6-tétrahydro 1-pyridine éthanol et son oxalate.

9. Procédé de préparation des composés de formule (I) tels que définis à l'une quelconque des revendications 1 à 8, ainsi que de leurs sels, caractérisé en ce que l'on condense la N-benzyl 4-pipéridinone avec un composé de formule (II):

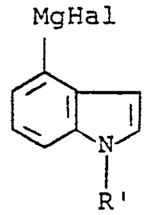

(II)

dans laquelle Hal représente un atome d'halogène et R' un radical alkyle renfermant de 1 à 8 atomes de carbone ou un radical aralkyle renfermant de 7 à 12 atomes de carbone, pour obtenir un composé de formule ($I_A$):

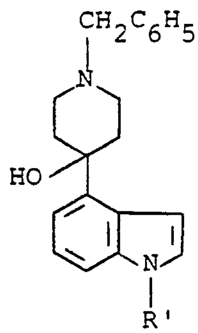

($I_A$)

puis, si désiré, soumet le composé de formule ($I_A$) à l'action d'un agent de clivage du groupement benzyle porté par l'azote du groupement pipéridinyle, pour obtenir un composé de formule ($I_B$):

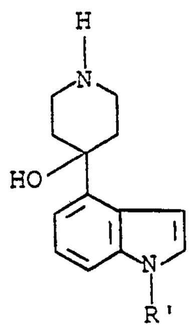

($I_B$)

que l'on soumet, si désiré, à l'action d'un agent de déshydratation, pour obtenir un composé de formule ($I_C$):

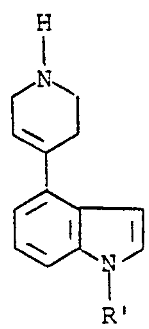

($I_C$)

ou bien, dans le cas où R' représente un groupe benzyle, soumet le composé de formule ($I_A$), à l'action d'un agent de clivage sélectif du groupement benzyle porté par le noyau indole, pour obtenir un composé de formule ($I_D$):

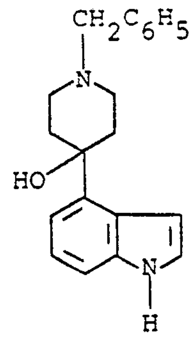

$(I_D)$

que l'on soumet, si désiré, à l'action d'un agent de clivage du groupement benzyle porté par le noyau pipéridinyle, pour obtenir un composé de formule $(I_F)$:

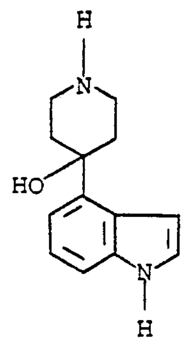

$(I_F)$

puis soumet, si désiré, chacun des composés obtenus répondant à la formule (I) dans laquelle Z représente un atome d'hydrogène, à l'action d'un agent capable d'introduire un radical Z', Z' ayant la même signification que Z, à l'exception d'hydrogène, pour obtenir les composés de formule (I) correspondants dans lesquels l'atome d'azote du noyau pipéridinyle porte un radical Z', et, si désiré, l'on soumet chacun des composés répondant à la formule (I) dans laquelle Z est différent d'un atome d'hydrogène et dans laquelle R représente un atome d'hydrogène à l'action d'un agent d'alkylation ou d'aralkylation pour obtenir les composés de formule (I) dans laquelle R représente un radical alkyle renfermant de 1 à 8 atomes de carbone ou un radical aralkyle renfermant de 7 à 12 atomes de carbone et, si désiré,

- soit l'on soumet l'un ou l'autre des composés obtenus répondant à la formule (I) dans laquelle b représente un radical hydroxyle à l'action d'un agent d'éthérification pour obtenir les composés de formule (I) correspondants dans laquelle b représente un radical alkoxy renfermant de 1 à 8 atomes de carbone,
- soit l'on soumet l'un ou l'autre des composés obtenus répondant à la formule (I) dans laquelle b représente un radical hydroxyle à l'action d'un agent de déshydratation pour obtenir les composés de formule (I) correspondants dans laquelle a et b forment ensemble une double liaison carbone-carbone,
- soit l'on soumet l'un ou l'autre des composés obtenus répondant à la formule (I) dans laquelle a et b forment ensemble une double liaison carbone-carbone, à l'action d'un agent de réduction, pour obtenir les composés de formule (I) correspondants dans laquelle a et b représentent chacun un atome d'hydrogène,
- soit l'on soumet l'un ou l'autre des composés obtenus répondant à la formule (I) dans laquelle b représente un radical hydroxyle, à l'action d'un agent de clivage du groupement hydroxyle, pour obtenir les composés de formule (I) correspondants dans laquelle a et b représentent chacun un atome d'hydrogène

et, si désiré, soumet chacun des composés de formule (I) ainsi obtenus, à l'action d'un agent d'halogénation susceptible d'introduire un atome d'halogène en 3 du noyau indole et hydrolyse le dérivé halogéné ainsi obtenu pour préparer les composés de formule (I) comportant un groupement 2-oxo et si désiré, soumet chacun des composés de formule (I) obtenus précédemment, à l'action d'un acide pour en former le sel.

10. Procédé selon la revendication 9, caractérisé en ce que l'introduction du radical Z' est réalisée:

- grâce à une réaction d'hydroxyméthylation suivie d'une réduction du méthylol intermédiaire formé, lorsque Z' est un radical méthyle,
- au moyen d'un halogénure Z'-Hal lorsque Z' est un radical alkyle renfermant de 1 à 8 atomes de carbone, un radical alkényle renfermant de 2 à 8 atomes de carbone, un radical hydroxyalkyle renfermant de 2 à 8 atomes de carbone, un radical cyanoalkyle renfermant de 3 à 8 atomes de carbone, un radical aralkyle renfermart de 7 à 12 atomes de carbone, un radical cycloalkylalkyle renfermant de 4 à 12 atomes de carbone, un groupement

$$-(CH_2)_n-O-Ar \quad ou \quad -(CH_2)_n-\underset{\underset{O}{\|}}{C}-Ar,$$

- au moyen d'un époxyde de formule 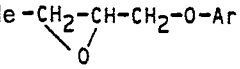 dans laquelle Ar a la signification déjà indiquée, lorsque Z' est le groupement

$$-CH_2-\underset{OH}{\underset{|}{CH}}-CH_2-O-Ar,$$

- au moyen de l'acrylonitrile lorsque Z' est un radical cyanométhyle.

11. A titre de médicaments, les produits de formule (I), tels que définis à l'une quelconque des revendications 1 à 7 et leurs sels d'addition avec les acides pharmaceutiquement acceptables.

12. A titre de médicaments, les produits tels que définis à la revendication 8.

13. Les compositions pharmaceutiques contenant à titre de principe actif, l'un au moins des médicaments tels que définis par la revendication 11 ou 12.

**Patentansprüche**

1. Verbindungen der Formel (I)

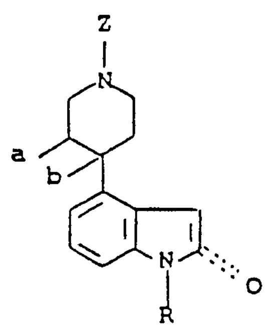

(I)

worin
R für ein Wasserstoffatom, einen Alkylrest mit 1 bis 8 Kohlenstoffatomen oder einen Aralkylrest mit 7 bis 12 Kohlenstoffatomen steht,
Z ein Wasserstoffatom, einen Alkylrest mit 1 bis 8 Kohlenstoffatomen, einen Alkenylrest mit 2 bis 8 Kohlenstoffatomen, einen Cyanoalkylrest mit 3 bis 8 Kohlenstoffatomen, einen Hydroxyalkylrest mit 2 bis 8 Kohlenstoffatomen, einen Rest der Formel $-(CH_2)_n-O-Ar$, der Formel $-(CH_2)_n-\underset{\underset{O}{\|}}{C}-Ar$ oder der Formel $-CH_2-\underset{OH}{\underset{|}{CH}}-CH_2-O-Ar$ wiedergibt, worin n eine ganze Zahl von 2 bis 8 bedeutet und Ar einen Phenyl-, Naphthyl-, Pyridyl-, Thienyl-, Thiazolylrest wiedergibt, der gegebenenfalls substituiert ist durch einen oder mehrere Reste, ausgewählt unter den Alkylresten mit 1 bis 5 Kohlenstoffatomen, den Alkoxyresten mit 1 bis 5 Kohlenstoffatomen, den Halogenatomen, der Hydroxygruppe, den Hydroxyalkylresten mit 1 bis 5 Kohlenstoffatomen, den Alkenyl-, Alkenyloxy-, Alkinyl-, Alkinyloxy-, Nitro-, Amino-, Trifluormethylresten, oder Z einen Aralkylrest mit 7 bis 12 Kohlenstoffatomen wiedergibt, der gegebenenfalls substituiert ist durch einen oder mehrere Reste, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl, Trifluormethoxy-, Nitro- und Aminogruppen, oder Z für einen Cycloalkalkylrest mit 4 bis 12 Kohlenstoffatomen steht und
entweder a und b jeweils für ein Wasserstoffatom stehen
oder a für ein Wasserstoffatom steht und b eine Hydroxylgruppe oder eine Alkoxygruppe mit 1 bis 8 Kohlenstoffatomen wiedergibt,
oder a und b gemeinsam eine Kohlenstoff-Kohlenstoff-Doppelbindung bilden und die gestrichelte 2-Oxo-Gruppe an dem Indolring die etwaige Anwesenheit der 2-Oxo-Gruppe anzeigt, wobei, wenn die 2-Oxo-Gruppe an dem Indolring vorliegt, die Kohlenstoff-Kohlenstoff-Doppelbindung des Indolrings nicht vorhanden ist, sowie die Additionssalze der Verbindungen der Formel (I) mit Säuren.

2. Verbindungen der Formel (I) gemäß Anspruch 1, worin R für ein Wasserstoffatom oder einen Alkylrest mit 1 bis 4 Kohlenstoffatomen steht, sowie deren Additionssalze mit Säuren.

3. Verbindungen der Formel (I) gemäß den Ansprüchen 1 und 2, worin Z für ein Wasserstoffatom oder einen

Alkylrest mit 1 bis 4 Kohlenstoffatomen oder einen Rest $-(CH_2)_n-O-Ar$ oder $-CH_2-\underset{OH}{CH}-CH_2-O-Ar$ steht, worin n eine ganze Zahl von 2 bis 4 bedeutet und Ar einen gegebenenfalls substituierten Phenyl- oder Thienylrest wiedergibt, sowie deren Additionssalze mit Säuren.

4. Verbindungen der Formel (I) gemäß den Ansprüchen 1 bis 3, worin Z für einen Rest $-CH_2-\underset{OH}{CH}-CH_2-O-Ar$ steht, worin Ar einen durch eine Propenyloxygruppe substituierten Phenylrest wiedergibt, sowie deren Additionssalze mit Säuren.

5. Verbindungen der Formel (I) gemäß den Ansprüchen 1 bis 4, worin a und b jeweils ein Wasserstoffatom wiedergeben, sowie deren Additionssalze mit Säuren.

6. Verbindungen der Formel (I) gemäß einem der Ansprüche 1 bis 4, worin a und b gemeinsam eine Kohlenstoff-Kohlenstoff-Doppelbindung bilden, sowie deren Additionssalze mit Säuren.

7. Verbindungen der Formel (I) gemäß einem der Ansprüche 1 bis 4, worin a ein Wasserstoffatom bedeutet und b für einen Hydroxylrest oder eine Methoxygruppe steht, sowie deren Additionssalze mit Säuren.

8. Eine der Verbindungen der Formel (I) mit den folgenden Bezeichnungen:
1,3-Dihydro-4-[1-[2-hydroxy-3-[2-(2-propenyloxy)-phenoxy]-propyl]-1,2,3,6-tetrahydro-4-pyridinyl-]-2H-indol-2-on und dessen Hydrochlorid;
4-(4-Methoxy-4-piperidinyl)-1H-Indol und dessen Fumarat;
4-(1H-Indol-4-yl)-α-[[2-(2-propenyloxy)-phenoxy]-methyl]-1,2,3,6-tetrahydro-1-pyridin-ethanol und dessen Oxalat.

9. Verfahren zur Herstellung der Verbindungen der Formel (I) gemäß einem der Ansprüche 1 bis 8 sowie von deren Salzen, dadurch gekennzeichnet, daß man N-Benzyl-4-piperidinon mit einer Verbindung der Formel (II)

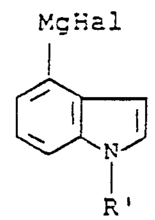

(II)

worin Hal für ein Halogenatom steht und R' einen Alkylrest mit 1 bis 8 Kohlenstoffatomen oder einen Aralkylrest mit 7 bis 12 Kohlenstoffatomen wiedergibt, kondensiert, um zu einer Verbindung der Formel ($I_A$)

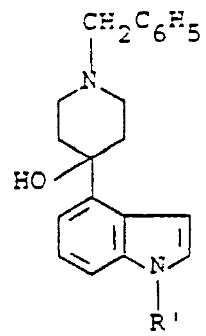

($I_A$)

zu gelangen, hiernach gewünschtenfalls die Verbindung der Formel ($I_A$) der Einwirkung eines Mittels zur Abspaltung der an dem Stickstoff der Piperidinylgruppe vorliegenden Benzylgruppe unterzieht, um zu einer Verbindung der Formel ($I_B$)

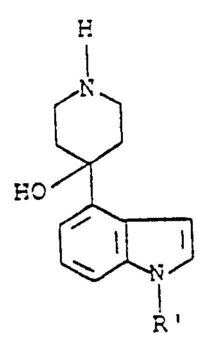

($I_B$)

zu gelangen, die man gewünschtenfalls der Einwirkung eines Dehydratationsmittels unterzieht, um zu einer Verbindung der Formel ($I_C$)

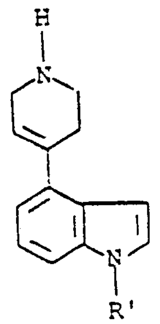

$(I_C)$

zu gelangen, oder, wenn R' für eine Benzylgruppe steht, man die Verbindung der Formel ($I_A$) der Einwirkung eines Mittels zur selektiven Abspaltung der an dem Indolring vorliegenden Benzylgruppe unterzieht, um zu einer Verbindung der Formel ($I_D$)

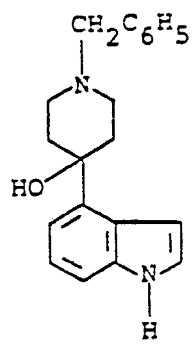

$(I_D)$

zu gelangen, die man gewünschtenfalls der Einwirkung eines Mittels zur Abspaltung der an dem Piperidinylring vorliegenden Benzylgruppe unterzieht, um zu einer Verbindung der Formel ($I_F$)

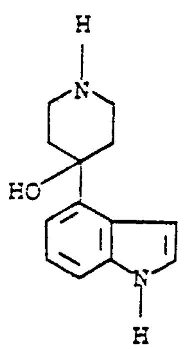

$(I_F)$

zu gelangen, hiernach gewünschtenfalls eine jede der erhaltenen Verbindungen der Formel (I), worin Z für ein Wasserstoffatom steht, der Einwirkung eines Mittels unterzieht, das befähigt ist, einen Rest Z' einzuführen, wobei Z' die Bedeutung von Z mit Ausnahme von Wasserstoff besitzt, um zu den entsprechenden Verbindungen der Formel (I) zu gelangen, worin das Stickstoffatom des Piperidinylrings einen Rest Z' aufweist, und gewünschtenfalls eine jede der der Formel (I) entsprechenden Verbindungen, worin Z von einem Wasserstoffatom verschieden ist und worin R ein Wasserstoffatom bedeutet, der Einwirkung eines Alkylierungs- oder Aralkylierungsmittels unterzieht, um zu den Verbindungen der Formel (I) zu gelangen, worin R für einen Alkylrest mit 1 bis 8 Kohlenstoffatomen oder einen Aralkylrest mit 7 bis 12 Kohlenstoffatomen steht, und gewünschtenfalls

entweder die eine oder andere der erhaltenen Verbindungen der Formel (I), worin b für eine Hydroxylgruppe steht, der Einwirkung eines Veretherungsmittels unterzieht, um zu den entsprechenden Verbindungen der Formel (I) zu gelangen, worin b für eine Alkoxygruppe mit 1 bis 8 Kohlenstoffatomen steht,

oder die eine oder andere der erhaltenen Verbindungen der Formel (I), worin b für einen Hydroxylrest steht, der Einwirkung eines Dehydratationsmittels unterzieht, um zu den entsprechenden Verbindungen der Formel (I) zu gelangen, worin a und b gemeinsam eine Kohlenstoff-Kohlenstoff-Doppelbindung bilden,

oder die eine oder andere der erhaltenen Verbindungen der Formel (I), worin a und b gemeinsam eine Kohlenstoff-Kohlenstoff-Doppelbindung bilden, der Einwirkung eines Reduktionsmittels unterzieht, um zu den entsprechenden Verbindungen der Formel (I) zu gelangen, worin a und b jeweils ein Wasserstoffatom

bedeuten,
oder die eine oder andere der erhaltenen Verbindungen der Formel (I), worin b für eine Hydroxylgruppe steht, der Einwirkung eines Mittels zur Abspaltung der Hydroxylgruppe unterzieht, um zu den entsprechenden Verbindungen der Formel (I) zu gelangen, worin a und b jeweils für ein Wasserstoffatom stehen, und, gewünschtenfalls, eine jede der so erhaltenen Verbindungen der Formel (I) der Einwirkung eines Halogenierungsmittels unterzieht, das befähigt ist, ein Halogenatom in 3-Stellung des Indolrings einzuführen, und das so erhaltene Halogenderivat hydrolysiert, um zu den Verbindungen der Formel (I) zu gelangen, die eine 2-Oxo-Gruppe aufweisen, und gewünschtenfalls eine jede der vorstehend erhaltenen Verbindungen der Formel (I) der Einwirkung einer Säure unterzieht, um hieraus das Salz zu bilden.

10. Verfahren gemäß Anspruch 9, dadurch gekennzeichnet, daß die Einführung des Restes Z' durchgeführt wird:
mit Hilfe einer Hydroxymethylierungsreaktion, gefolgt von einer Reduktion des intermediär gebildeten Methylols, wenn Z' für eine Methylgruppe steht,
mit Hilfe eines Halogenids Z'-Hal, wenn Z' für einen Alkylrest mit 1 bis 8 Kohlenstoffatomen, einen Alkenylrest mit 2 bis 8 Kohlenstoffatomen, einen Hydroxyalkylrest mit 2 bis 8 Kohlenstoffatomen, einen Cyanoalkylrest mit 3 bis 8 Kohlenstoffatomen, einen Aralkylrest mit 7 bis 12 Kohlenstoffatomen, einen Cycloalkalkylrest mit 4 bis 12 Kohlenstoffatomen, eine Gruppe $-(CH_2)_n-O-Ar$ oder $-(CH_2)_n-\underset{\overset{\|}{O}}{C}-Ar$ steht,
mit Hilfe eines Epoxids der Formel $\underbrace{CH_2 - CH}_{O}-CH_2-O-Ar$, worin Ar die angegebene Bedeutung besitzt, wenn Z' die Gruppe $-CH_2-\underset{OH}{CH}-CH_2-O-Ar$ wiedergibt, mit Hilfe von Acrylnitril, wenn Z' einen Cyanomethylrest bedeutet.

11. Als Arzneimittel die Produkte der Formel (I) gemäß einem der Ansprüche 1 bis 7 und deren Additionssalze mit pharmazeutisch verträglichen Säuren.

12. Als Arzneimittel die Produkte gemäß Anspruch 8.

13. Pharmazeutische Zusammensetzungen, enthaltend als Wirkstoff zumindest eines der Arzneimittel gemäß Anspruch 11 oder 12.

## Claims

1. Compounds of formula (I):

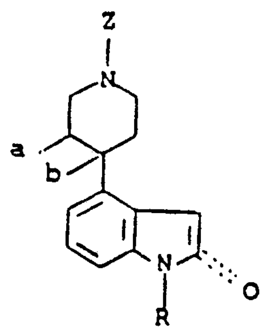

(I)

in which
R represents a hydrogen atom, an alkyl radical containing from 1 to 8 carbon atoms or an aralkyl radical containing from 7 to 12 carbon atoms,
Z represents a hydrogen atom, an alkyl radical containing from 1 to 8 carbon atoms, an alkenyl radical containing from 2 to 8 carbon atoms, a cyanoalkyl radical containing from 3 to 8 carbon atoms, a hydroxyalkyl radical containing from 2 to 8 carbon atoms, a radical of formula $-(CH_2)_n-O-Ar$, of formula $-(CH_2)_n-\underset{\overset{\|}{O}}{C}-Ar$, or of formula $-CH_2-\underset{OH}{CH}-CH_2-O-Ar$, in which n represents an integer which can vary from 2 to 8 and Ar represents a phenyl, naphthyl, pyridyl, thienyl, or thiazolyl radical optionally substituted by one or more radicals selected from the group formed by alkyl radicals containing from 1 to 5 carbon atoms, alkoxy radicals containing from 1 to 5 carbon atoms, halogen atoms, the hydroxy radical, hydroxyalkyl radicals containing from 1 to 5 carbon atoms, alkenyl, alkenyloxy, alkynyl, alkynyloxy, nitro, amino, trifluoromethyl radicals, or Z represents an aralkyl radical containing from 7 to 12 carbon atoms optionally substituted by one or more radicals selected from the group formed by alkyl radicals containing from 1 to 5 carbon atoms, alkoxy radicals containing from 1 to 5 carbon atoms, halogen atoms, hydroxyl, trifluoromethyl, trifluoromethoxy, nitro and amino radicals or Z represents a cycloalkylalkyl radical containing from 4 to 12 carbon atoms and,
- either a and b each represent a hydrogen atom,
or a represents a hydrogen atom and b represents a hydroxyl radical or an alkoxy radical containing from 1 to 8 carbon atoms,
- or a and b together form a double carbon-carbon bond and the 2-oxo group shown in dotted lines on the indole nucleus signifies the optional presence of a 2-oxo group, it being understood that when the 2-oxo group is present on the indole nucleus, the double carbon-carbon bond of the indole nucleus can no longer exist, as

well as the addition salts with acids of the compounds of formula (I).

2. Compounds of formula (I) as defined in claim 1 in which R represents a hydrogen atom or an alkyl radical containing from 1 to 4 carbon atoms, as well as their addition salts with acids.

3.- Compounds of formula (I), as defined in claims 1 and 2, in which Z represents a hydrogen atom or an alkyl radical containing from 1 to 4 carbon atoms or a $-(CH_2)_n$-C-Ar radical or a $-CH_2-\underset{OH}{CH}-CH_2-O-Ar$ in which n represents an integer which can vary from 2 to 4 and Ar is a phenyl or thienyl radical optionally substituted, as well as their addition salts with acids.

4. Compounds of formula (I), as defined in claims 1 to 3, in which Z represents a $-CH_2-\underset{OH}{CH}-CH_2-O-Ar$, Ar being a phenyl radical substituted by a propenyloxy group, as well as their addition salts with acids.

5. Compounds of formula (I), as defined in claims 1 to 4, in which a and b each represent a hydrogen atom, as well as their addition salts with acids.

6. Compounds of formula (I), as defined in claims 1 to 4, in which a and b together form a double carbon-carbon bond, as well as their addition salts with acids.

7. Compounds of formula (I), as defined in any one of claims 1 to 4, in which a represents a hydrogen atom and b represents a hydroxyl radical or a methoxy radical, as well as their addition salts with acids.

8. Any one of the compounds of formula (I) which have the following names:
- 1,3-dihydro 4-[-[2-hydroxy 3-[2-(2-propenyloxy) phenoxy]propyl] 1,2,3,6-tetrahydro 4-pyridinyl] 2H-indol-2-one and its hydrochloride;
- 4-(4-methoxy 4-piperidinyl) 1H-indole and its fumarate;
- 4-(1H-indol-4-yl) alpha-[[2-(2-propenyloxy) phenoxy]methyl] 1,2,3,6-tetrahydro 1-pyridine ethanol and its oxalate.

9. Process for the preparation of the compounds of formula (I) as defined in any one of claims 1 to 8, as well as their salts characterized in that N-benzyl 4-piperidinone is condensed with a compound of formula (II):

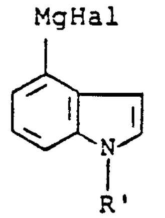

(II)

in which Hal represents a halogen atom and R' is an alkyl radical containing from 1 to 8 carbon atoms or an aralkyl radical containing from 7 to 12 carbon atoms to obtain a compound of formula ($I_A$):

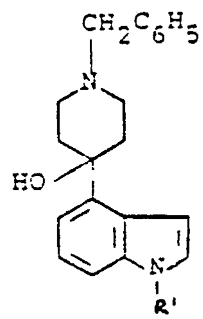

($I_A$)

then, if desired, the compound of formula ($I_A$) is submitted to the action of a cleavage agent of the benzyl group carried by the nitrogen of the piperidinyl group to obtain a compound of formula ($I_B$):

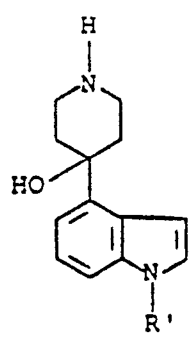

($I_B$)

which is submitted, if desired, to the action of a dehydration agent to obtain a compound of formula ($I_C$):

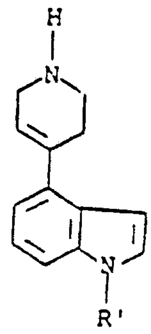

($I_C$)

or, in the case where R' represents a benzyl group, the compound of formula ($I_A$) is submitted to the action of a selective cleavage agent of the benzyl group carried by the indole nucleus, to obtain a compound of formula ($I_D$):

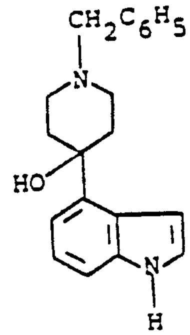

($I_D$)

which is submitted, if desired, to the action of a cleavage agent of the benzyl group carried by the piperidinyl nucleus to obtain a compound of formula ($I_F$):

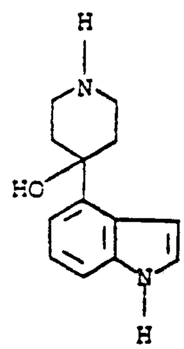

($I_F$)

then, if desired, each of the compounds obtained corresponding to formula (I) in which Z represents a hydrogen atom is submitted to the action of an agent able to introduce a radical Z', Z' having the same meaning as Z, with the exception of hydrogen, to obtain the corresponding compounds of formula (I) in which the nitrogen atom of the piperidinyl nucleus carries a radical Z', and, if desired, each of the compounds corresponding to formula (I) in which Z is different from a hydrogen atom and in which R represents a hydrogen atom is submitted to the action of an alkylation or an aralkylation agent to obtain the compounds of formula (I) in which R represents an alkyl radical containing from 1 to 8 carbon atoms or an aralkyl radical containing from 7 to 12 carbon atoms, and if desired,

- either one or the other of the compounds obtained corresponding to formula (I) in which b represents a hydroxyl radical is submitted to the action of an etherification agent to obtain the corresponding compounds of formula (I) in which b represents an alkoxy radical containing from 1 to 8 carbon atoms,
- or one or the other of the compounds obtained corresponding to formula (I) in which b represents a hydroxyl radical is submitted to the action of a dehydration agent to obtain the corresponding products of formula (I) in which a and b together form a double carbon-carbon bond,
- or one or the other of the compounds obtained corresponding to formula (I) in which a and b together form a double carbon-carbon bond is submitted to the action of a reduction agent to obtain the corresponding compounds of formula (I) in which a and b each represent a hydrogen atom,
- or one or the other of the products obtained corresponding to formula (I) in which b represents a hydroxyl

radical is submitted to the action of a cleavage agent of the hydroxyl group to obtain the corresponding compounds of formula (I) in which a and b each represent a hydrogen atom

and, if desired, each of the compounds of formula (I) so obtained, is submitted to the action of a halogenation agent able to introduce a halogen atom in position 3 of the indole nucleus and the halogenized derivative so obtained is hydrolyzed to prepare the compounds of formula (I) containing a 2-oxo group and if desired, each of the compounds of formula (I) obtained above is submitted to the action of an acid to form the salt.

10. Process according to claim 9, characterized in that the introduction of the radical Z' is carried out:

- by a hydroxymethylation reaction followed by the reduction of the intermediate methylol formed, when Z' is a methyl radical,
- by means of a halogenide Z'-Hal when Z' is an alkyl radical containing from 1 to 8 carbon atoms, an alkenyl radical containing from 2 to 8 carbon atoms, a hydroxyalkyl radical containing from 2 to 8 carbon radicals, a cyanoalkyl radical containing from 3 to 8 carbon atoms, an aralkyl radical containing from 7 to 12 carbon atoms, a cycloalkylalkyl radical containing from 4 to 12 carbon atoms, a group $-(CH_2)_n-O-Ar$ or

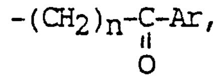

- by means of an epoxide of formula 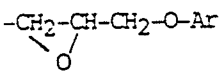 in which Ar has the meaning already given, when Z' is the group 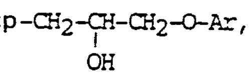
- by means of acrylonitrile when Z' is a cyanomethyl radical.

11. As medicaments, products of formula (I), as defined in any one of claims 1 to 7 and their addition salts with pharmaceutically acceptable acids.

12. As medicaments, products as defined in claim 8.

13. Pharmaceutical compositions containing at least one of the medicaments as defined in claim 11 or 12 as active principle.